# EUROPEAN PATENT APPLICATION

(11) **EP 0 827 946 A1**
(43) Date of publication of application: **11.03.1998**
(21) Application number: 96202461.8
(22) Date of filing: 04.09.1996
(51) Int. Cl.: C07C 57/72, A61K 31/19

(54) **Inhibitors for the differentiation of cells, in particular hepatic stellate cells**

(71) Applicant: VRIJE UNIVERSITEIT BRUSSEL, 1050 Brussel (BE)
(72) Inventor: Geerts, Albert Emmanuel Corneille, 1150 Brussel (BE); Niki, Toshiro, 1780 Wemmel (BE)
(74) Representative: Eveleens Maarse, Pieter

(57) **Abstract**

This invention relates to new compounds as inhibitors for the differentiation of cells, in particular hepatic stellate cells.

It was surprisingly found that new compounds of the following formula: 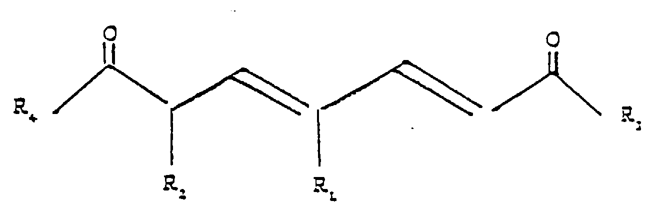 wherein:
R₁ and R₂ are independently selected from the group comprising of hydrogen, alkyl, hydroxy, amino and nitro;
R₃ is selected from the group comprising of halogen, alkyl, halogenalkyl, formyl, alkoxycarbonyl, acyl, hydroxy, alkoxy, acyloxy, glycosyloxy, amino, alkylamino mercapto, alkylthio, nitro, alkylnitro, epoxy and alkylepoxy; and
R₄ is selected from the group comprising of hydrogen; phenyl, p-benzoquinonyl or cumarinyl which may have at least one substituent selected from the group comprising of halogen, alkyl, halogenalkyl, formyl, alkoxycarbonyl, acyl, hydroxy, alkoxy, acyloxy, glycosyloxy, amino, alkylamino mercapto, alkylthio, nitro, alkylnitro, epoxy and alkylepoxy;
are effective as an active ingredient in the treatment of fibrosis.

## Description

Eukaryotic gene expression has been mainly studied in the context of transacting transcription factors and their interaction with regulatory cis-elements. Evidence is accumulating, however, that high order structure of chromatin plays an essential role in eukaryotic gene regulation. One major process by which chromatin structure is modulated is histone acetylation. Lysine residues in the N-terminal domain of histone molecules are reversibly acetylated, the level of which is determined by an equilibrium between histone acetyltransferase and deacetylase. It is thought that acetylation of lysine reduces the positive charge of the N-terminal tail domain of histone to weaken their interaction with DNA in the nucleosome. Importance of histone acetylation was high-lighted by the recent findings that the genes encoding histone acetyltransferase and deacetylase are homologous to yeast gene regulators, Gcn5p and Rpd3p, respectively.

Although many genetic, biochemical, and immunological studies have shown that actively transcribed genes are generally associated with hyperacetylated histones, the exact biologic significance of histone acetylation in mammalian cells is still unclear. In this respect use of specific pharmacologic inhibitors may provide further insight. Trichostatin A, an inhibitor of histone deacetylase has been shown to cause G1 and G2 phase cell cycle arrest in fibroblasts, to induce differentiation in erythroleukemia cells, and to inhibit sisor ras-induced transformation of NIH3T3 cells.

Our laboratory has been investigating the mechanisms underlying liver fibrosis, especially with the aim of developing new therapeutic agents. In liver fibrosis, hepatic stellate cells are the major cell type responsible for the excessive accumulation of extracellular matrix proteins. In normal liver, these cells are liver-specific pericytes which serve as major vitamin A storage sites and show little proliferative activity. In injured livers, however, stellate cells change their phenotype and differentiate into myofibroblasts. During this transition they lose their vitamin A-containing fat-droplets, and acquire the capacity to proliferate and to produce excessive quantities of extracellular matrix proteins. Furthermore, growing evidence indicates that active myofibroblasts are central to fibrogenesis in other organs, such as skin, lung and kidney. Thus inhibition of myofibroblastic differentiation appears to be an attractive common target for therapeutic intervention in fibroproliferative diseases.

Surprisingly it was found that new compounds which were structurally related with Trichostatin A were useful as inhibitors for differentiation of cells, in particular hepatic stellate cells.

These compounds have the following general structural formula: 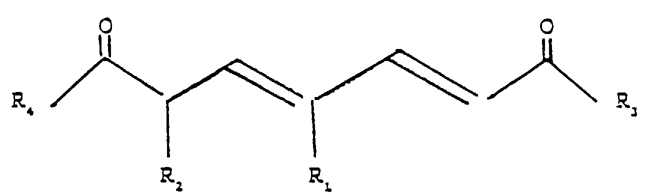
R₁ and R₂ are independently selected from the group comprising of hydrogen, C1-C6 alkyl, hydroxy, amino and nitro;
R₃ is selected from the group comprising of halogen, alkyl, halogenalkyl, formyl, alkoxycarbonyl, acyl, hydroxy, alkoxy, acyloxy, glycosyloxy, amino, alkylamino mercapto, alkylthio, nitro, alkylnitro, epoxy and alkylepoxy; and
R₄ is selected from the group comprising of hydrogen; phenyl, p-benzoquinonyl or cumarinyl which may have at least one substituent selected from the group comprising of halogen, alkyl, halogenalkyl, formyl, alkoxycarbonyl, acyl, hydroxy, alkoxy, acyloxy, glycosyloxy, amino, alkylamino mercapto, alkylthio, nitro, alkylnitro, epoxy and alkylepoxy.

Using conventional synthesis methods compound A was prepared. Compound A is a compound of the above given formula wherein R₁ = hydrogen, R₂ = methyl, R₃ = chlorine and R₄ = phenyl. The effect of this compound A on the synthesis of collagens type I and III was tested using hepatic stellate cells from adult Wistar rats.

After isolation cells were suspended in Dulbecco's modified Eagle's medium supplemented with 10 % fetal calf serum, 100 U/ml penicillin, and 100 µg/ml streptomycin, and cultured at 37°C in a humidified atmosphere with 5 % CO₂ and 95 % air.

At day 3 cells were exposed to compound A (1-100 nmol/L) for 24 h. During the subsequent 24 h cells were metabolically labeled with 25 µCi/ml of Trans ³⁵S-label (specific activity of ³⁵S-methionine > 1,000 Ci/mmol, ICN Biomedicals, Costa Mesa CA) while exposure to the compounds was continued. After labelling medium was collected, and subjected to immunoprecipitation using antibodies against collagens type I and III. The precipitates were fractionated by SDS-PAGE and radioactivity of specific bands were measured by the PhosphorImaging technology. For the effect at mRNA level, cells were exposed to 100 nmol/L compound A for 24 h. RNA was then extracted and analyzed by Northern hybridization analysis.

Table 1 shows the results which were expressed as percentage value relative to the control culture.

**Table 1**

| | 100 nM | 10 nM | 1 nM |
|---|---|---|---|
| collagen I | 45.0±6.4 | 78.3±5.6 | 98.4±10.2 |
| collagen III | 39.0±10.6 | 80.3±21.8 | 83.8±24.1 |

These results show that compound A is an active ingredient for the preparation of a pharmaceutical composition for the treatment of fibrosis, in particular liver fibrosis. Similar analogue compounds - compounds of claim 1 - will give similar results.

## Claims

1. Compounds of the formula: 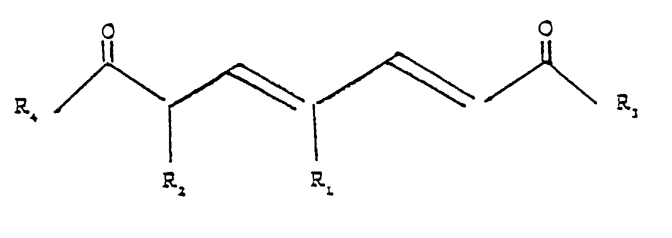 wherein:
R₁ and R₂ are independently selected from the group comprising of hydrogen, alkyl, hydroxy, amino and nitro;
R₃ is selected from the group comprising of halogen, alkyl, halogenalkyl, formyl, alkoxycarbonyl, acyl, hydroxy, alkoxy, acyloxy, glycosyloxy, amino, alkylamino mercapto, alkylthio, nitro, alkylnitro, epoxy and alkylepoxy; and
R₄ is selected from the group comprising of hydrogen; phenyl, p-benzoquinonyl or cumarinyl which may have at least one substituent selected from the group comprising of halogen, alkyl, halogenalkyl, formyl, alkoxycarbonyl, acyl, hydroxy, alkoxy, acyloxy, glycosyloxy, amino, alkylamino mercapto, alkylthio, nitro, alkylnitro, epoxy and alkylepoxy.

2. Compound as claimed in claim 1 for use in the treatment of fibrosis, in particular liver fibrosis.

3. Pharmaceutical compositions comprising compounds of claim 1 and a suitable excipient.

4. Use of compounds of claim 1 as active ingredient for the preparation of a pharmaceutical composition for the treatment of fibrosis, in particular liver fibrosis.

5. Method of treatment of fibrosis by using as active ingredient an effective amount of a compound of claim 1.
